# EUROPEAN PATENT APPLICATION

(11) **EP 4 748 350 A2**
(43) Date of publication of application: **27.05.2026**
(21) Application number: 26161379.8
(22) Date of filing: 06.03.2024
(51) Int. Cl.: A61F 2/38

(54) **METHOD OF DESIGNING TIBIAL BEARING INSERT, KIT INCLUDING TIBIAL BEARING INSERT DESIGNED BY SUCH METHOD AND COMPUTER-READABLE MEDIUM**

(30) Priority: 06.03.2023 US 202363450154 P
(62) Divisional of application: 24161718.2
(71) Applicant: Howmedica Osteonics Corp., Mahwah, NJ 07430 (US)
(72) Inventor: SERVIDIO, Damon J., TOWACO, 07082 (US)
(74) Representative: Regimbeau

(57) **Abstract**

A method of designing a tibial bearing insert includes: identifying a radial arc centerline axis of a tibial bearing insert, the radial arc centerline axis passing through an apex of a lateral radial arc of the tibial bearing insert and an apex of a medial radial arc of the tibial bearing insert, both in a first transverse plane, the lateral and medial radial arcs representing lines of minimum thickness of the tibial bearing insert between an anterior side and a posterior side of the tibial bearing insert on an initial articular surface of the tibial bearing insert; aligning a center axis of a femoral flexion radius of a femoral component with the radial arc centerline axis such that the center axis and the radial arc centerline axis are coincident with a first coronal plane; while keeping the center axis in the first coronal plane, aligning the femoral component with the tibial bearing insert by positioning the femoral component such that an inferior most point of a circle representative of the femoral flexion radius and centered on the center axis defines a predetermined minimum thickness of the tibial bearing insert; and subsequent to aligning the center axis and aligning the femoral component with the tibial bearing insert, modifying a curvature of the initial articular surface of the tibial bearing insert between the anterior side and the posterior side to a modified articular surface that corresponds to a portion of the circle.

## Description

### CROSS-REFERENCE TO RELATED APPLICATION

This application claims the benefit of the filing date of U.S. Provisional Patent Application No. 63/450,154, filed March 6, 2023, the disclosure of which is hereby incorporated by reference herein in its entirety.

### BACKGROUND

Two main objectives of Total Knee Arthroplasty ("TKA") are the relief of pain caused by osteoarthritis and the restoration of function, or kinematics of the knee. With respect to restoring the function of the knee, range of motion and overall stability of the joint are key elements that contribute to the overall success and longevity of the procedure and overall patient satisfaction.

Knee stability is important throughout the entire range of motion, but particularly in mid-flexion, a phase during gait where an unstable knee becomes very noticeable to the patient especially during stair descending, walking down a steep incline or stepping off a curb. Important factors that contribute to joint stability are surgery related. For example, such factors may include joint balancing (degree of ligament and/or soft tissue tightness or laxity) as well as the design of the implant, in particular tibiofemoral articular surface congruency. The greater the congruency between the femur and the insert articular geometry, the greater the joint stability. A gross analogy is that of a ball and socket joint in which both components have nearly equal radius values. In such a joint the high degree of congruency results in an inherently very stable joint. By contrast, in a ball and socket joint in which the radius value of the socket is significantly larger than the radius value of the ball, the result is diminished stability since the ball is less restricted within the socket. The same applies to the sagittal and coronal curvatures of the femoral condylar geometry with respect to the mating and complementary geometry of the tibial plateau.

A scenario that is commonly seen in TKA is a size mismatch between the femoral implant and the tibial implant. In general, the femoral implant is selected for the optimum fit in terms of anterior-posterior and medial-lateral bone coverage on the resected femur. Likewise, the tibial baseplate is selected for the optimum fit in terms of anterior-posterior and medial-lateral bone coverage on the resected tibia. Often, the femur and tibia implant sizes are not the same. Thus, for example, a size 4 baseplate and by extension its corresponding bearing insert has to work with a size 3 or 5 femoral component. Sometimes the size disparity may be up to two sizes. This creates an articular interface conundrum since multiple size compatibility is a clinical requirement expected by physicians.

Optimization of the tibiofemoral articular congruency is limited in implant systems designed to allow multiple size compatibility. For example, in a scenario where a tibial baseplate of a given size must be compatible with femoral components two sizes larger and two sizes smaller, a size 2 femoral component must work with a size 4 tibial baseplate. And, the size 4 tibial baseplate must also work with a size 6 femoral component. Therefore, the articulation of a size 2 femur is mismatched with respect to the baseplate by four sizes since the same baseplate has to accommodate a size 6 femur.

Moreover, other factors may also lead to instability in the knee. Specifically, soft tissue in a healthy knee typically includes a posterior ligament and an anterior ligament that cross each other to form an X, with the anterior cruciate ligament in front of the knee and the posterior cruciate ligament in the back of the knee. The cruciate ligaments control anterior and posterior motion of the normal knee. During primary knee replacement surgery the anterior cruciate ligament is removed which may lead to increased forward and backward motion during normal activity, such forward and backward motion leading to instability.

Thus, a need exists to provide joint implant designs with improved post-surgical stability while simultaneously providing a practical solution to achieve such results.

### BRIEF SUMMARY

According to the invention, there is provided a method of designing a tibial bearing insert as defined in claim 1, as well as a kit comprising tibial bearing inserts designed in accordance with such a method and a non-transitory, computer-readable medium for causing a computer system to carry out such a method. Preferred, optional features of embodiments are defined in the corresponding dependent claims.

In one aspect, the present disclosure contemplates a baseplate system having a single common locking interface compatible throughout a size range of the system components. The common locking interface advantageously enables each femoral component to have a dedicated femur specific tibial insert designed with an optimized size-on-size articular geometry to be assembled to any baseplate, regardless of the size of the tibia. For example, a patient with a size 3 femoral component and a size 5 tibia would receive a size 3 femur-specific tibial insert assembled to a size 5 tibial baseplate sized for the tibia. In another example, a patient with a size 3 femoral component but with a size 2 tibia would also receive the size 3 femur-specific tibial insert but would have the tibial insert assembled to a size 2 tibial baseplate. This prevents articular congruency from being compromised by tibia size selection. Accordingly, in this novel approach, one femur-specific tibial bearing insert accommodates multiple tibial baseplate sizes.

In another aspect, the present disclosure contemplates a system with a baseplate, a bearing insert and a locking mechanism based on a fastener such as a rotating latch that is adjustable from a first unlocked position to a second locked position and vice versa. The latch advantageously allows the bearing insert to be easily and quickly unlocked and removed from the baseplate. Further, the latch facilitates this locking and unlocking function without causing damage to the baseplate. This is particularly useful in revision surgeries where a tibial insert is replaced due to wear or a change in constraint level in an otherwise well-fixed tibial baseplate since it is important not to damage the baseplate during the explantation process of the tibial insert.

In one aspect, the present disclosure contemplates a modular implant system. In a first example of a first embodiment, a modular implant system includes a baseplate, a bearing insert and a latch. The baseplate is adapted to receive the bearing insert. The bearing insert includes an articular surface and a plate-contacting surface opposite the articular surface. The latch is adapted for disposal through a hole in the bearing insert. When the plate-contacting surface of the bearing insert is disposed on the baseplate and the latch is disposed through the hole in the bearing insert, the latch is rotatable from a first position to a second position. The bearing insert is removable from the baseplate when the latch is in the first position and the bearing insert is held in place on the baseplate when the latch is in the second position.

With continued reference to the first embodiment, in a second example, the system may be arranged such that the latch of the first example may include a length with a shaft portion and a tab portion extending from the shaft portion. The tab portion may have an elongate dimension angled relative to the length of the latch and the elongate dimension may be longer than a maximum diameter of the shaft portion. In a third example, the second example of the system may be arranged such that the hole of the bearing insert includes an upper portion extending from the articular surface, a lower portion extending from the plate-contacting surface and a central portion therebetween. The central portion of this example may have a smaller cross-sectional dimension than the upper and lower portions. In a fourth example, the system of the third example may be arranged such that the upper and lower portions of the hole include concave wall surfaces and the central portion includes opposing wall sections projecting relative to the concave wall surfaces. The tab portion of the latch may fit within the central portion when elongate side walls of the tab portion are aligned with surfaces of the opposing wall sections of the central portion. In a fifth example, the system of the fourth example may be arranged such that when the latch is disposed in the hole, the opposing wall sections prevent removal of the latch from the bearing insert when the tab portion of the latch is disposed in the lower portion of the hole and the elongate side walls of the tab portion are not aligned with the surfaces of the opposing wall sections of the central portion. In a sixth example, the system of any one of the first through fifth examples may be arranged such that the baseplate includes a first protrusion defining a first recess thereunder such that a second protrusion of the bearing insert is slidable into the first recess to partially prevent sliding of the bearing insert relative to the baseplate. In a seventh example, the system of any one of the first through sixth examples may be arranged such that the latch has a length approximately equal to the hole of the bearing insert. In an eighth example, the system of any one of the first through seventh examples may be arranged such that the baseplate is one of a plurality of baseplates, each baseplate of the plurality of baseplates having a different size with respect to the others. In a ninth example, the system of any one of the first through eighth examples may be arranged such that the bearing insert is one of a plurality of bearing inserts, each bearing insert of the plurality of bearing inserts having a different size with respect to the others.

In a first example of a second embodiment, a modular implant system includes a baseplate, a bearing insert having a medial articular surface shaped to be congruent with a medial femoral condyle, and a fastener. The fastener may be disposed in the bearing insert and is adjustable between an unlocked and a locked position to control whether the bearing insert is irremovable from the baseplate.

With continued reference to the second embodiment, a second example of the system may be the first example where the fastener is a latch. In a third example, the medial articular surface of the bearing insert of any one of the first and second examples may be determined according to a method including: identifying a radial arc centerline axis of the bearing insert, the radial arc centerline axis passing through an apex of a lateral radial arc of the bearing insert and an apex of a medial radial arc of the bearing insert, both in a first transverse plane, the lateral and medial radial arcs representing lines of minimum thickness of the bearing insert between an anterior side and a posterior side of the bearing insert on an initial articular surface of the bearing insert; aligning a center axis of a femoral flexion radius of a femoral component with the radial arc centerline axis such that the center axis and the radial arc centerline axis are coincident with a first coronal plane; while keeping the center axis in the first coronal plane, aligning the femoral component with the bearing insert by positioning the femoral component such that an inferior most point of a circle representative of the femoral flexion radius and centered on the center axis defines a predetermined minimum thickness of the bearing insert; and subsequent to aligning the center axis and aligning the femoral component with the bearing insert, modifying a curvature of the initial articular surface of the bearing insert between the anterior side and the posterior side to correspond to a portion of the circle.

In another aspect, the present disclosure relates to a kit. In a first example of a first embodiment, the kit includes a first baseplate, a first bearing insert, a plurality of additional baseplates or a plurality of additional bearing inserts, and a latch. The latch is adapted to control engagement between any combination of baseplate and bearing insert from among the first baseplate, the plurality of additional baseplates, the first bearing insert and the plurality of additional bearing inserts. The latch performs this function of controlling engagement between a bearing insert and a baseplate while the latch is disposed in the bearing insert that is used for the engagement. When the kit includes the plurality of additional baseplates, the first baseplate and the plurality of additional baseplates are adapted for attachment to the first bearing insert. And, when the kit includes the plurality of additional bearing inserts, the first bearing insert and the plurality of additional bearing inserts are adapted for attachment to the first baseplate.

With continued reference to the first embodiment, in a second example, the kit of the first example may be arranged such that the latch includes a shaft and a distal tab, the latch being rotatable when fully disposed in one of the first bearing insert and the plurality of additional bearing inserts to control whether the distal tab is held in place by a protrusion of one of the first baseplate and the plurality of additional baseplates. Further, the one of the first baseplate and the plurality of additional baseplates and the one of the first bearing insert and the plurality of additional bearing inserts may be held together when the distal tab is held in place by the protrusion. In a third example, the kit of one of the first and second examples may be arranged such that each bearing insert of the first bearing insert and the plurality of additional bearing inserts includes a stepped hole adapted to receive the latch. The latch may have a shape such that the latch is fully disposable within the stepped hole. In a fourth example, the kit of any one of the first through third examples may be arranged such that the latch is one of a plurality of latches included in the kit, each latch of the plurality of latches being disposed in one of the first bearing insert and the plurality of additional bearing inserts. In a fifth example, the kit of any one of the first through fourth examples may be arranged such that each baseplate of the first baseplate and the plurality of additional baseplates also includes a proximal surface and a posterior recess on a posterior-side of the proximal surface. The posterior recess may be sized to receive a protrusion of any one of the first bearing insert and the plurality of additional bearing inserts. In a sixth example, the kit of the fifth example may be arranged such that each baseplate of the first baseplate and the plurality of additional baseplates may also include an anterior recess on the proximal surface sized to receive part of the latch when the latch is in a locked position. Each baseplate of the first baseplate and the plurality of additional baseplates may have a first dimension, i.e., the same dimension, between the posterior recess and the anterior recess. In a seventh example, the kit of any one of the fifth and sixth examples may be arranged such that each baseplate of the first baseplate and the plurality of additional baseplates also includes a posterior abutment and an anterior abutment on the proximal surface. An abutment distance between the posterior abutment and the anterior abutment may be the same for each baseplate. In an eighth example, the kit of any one of the first through seventh examples may be arranged such that the kit includes both the plurality of additional baseplates and the plurality of additional bearing inserts.

In another aspect, the present disclosure relates to a method of assembling an insert and a baseplate. In a first example of a first embodiment, the method includes steps as follows: placing an insert onto a baseplate such that an articular surface of the insert faces away from the baseplate; and rotating a latch disposed in a hole in the insert from an unlocked position into a locked position such that a tab portion of the latch enters a recess under a protrusion on an insert-facing surface of the baseplate, thereby preventing the insert from being removable from the baseplate while the latch is in the locked position.

With continued reference to the first embodiment, in a second example, placing the insert onto the baseplate as described in the first example may also include advancing a posterior protrusion of the insert into a posterior recess of the baseplate. In such example, the posterior recess may be defined by an anterior facing protrusion on the baseplate. In a third example, the method of any one of the first and second examples may include inserting the latch into the hole in the insert prior to the rotating step. In a fourth example, inserting the latch through the hole according to the method of the third example may also include orienting the latch into a first rotational position prior to inserting the latch, the hole being shaped such that the latch is only insertable from an external location when in the first rotational position. In a fifth example, the method of any one of the first through fourth examples may include selecting, prior to the placing step, the insert from among a plurality of inserts having different sizes. In a sixth example, the method of any one of the first through fifth examples may include selecting, prior to the placing step, the baseplate from among a plurality of baseplates having different sizes.

In yet another aspect, the present disclosure relates to a method of designing a tibial bearing insert. In a first example of a first embodiment of the method, the method includes: identifying a radial arc centerline axis of a tibial bearing insert, the radial arc centerline axis passing through an apex of a lateral radial arc of the tibial bearing insert and an apex of a medial radial arc of the tibial bearing insert, both in a first transverse plane, the lateral and medial radial arcs representing lines of minimum thickness of the tibial bearing insert between an anterior side and a posterior side of the tibial bearing insert on an initial articular surface of the tibial bearing insert; aligning a center axis of a femoral flexion radius of a femoral component with the radial arc centerline axis such that the center axis and the radial arc centerline axis are coincident with a first coronal plane; while keeping the center axis in the first coronal plane, aligning the femoral component with the tibial bearing insert by positioning the femoral component such that an inferior most point of a circle representative of the femoral flexion radius and centered on the center axis defines a predetermined minimum thickness of the tibial bearing insert; and subsequent to aligning the center axis and aligning the femoral component with the tibial bearing insert, modifying a curvature of the initial articular surface of the tibial bearing insert between the anterior side and the posterior side to a modified articular surface that corresponds to a portion of the circle.

With continued reference to the first embodiment, in a second example, the method of the first example may include identifying the apex of the lateral radial arc and the apex of the medial radial arc by determining a location on the respective radial arcs that is furthest from a central anterior-posterior axis through the tibial bearing insert. In a third example, the method of any one of the first and second examples may include: selecting a baseplate from among a plurality of baseplates having different sizes; and attaching the tibial bearing insert onto the baseplate such that the modified articular surface of the tibial bearing insert faces away from the baseplate. In a fourth example, aligning the femoral flexion radius of a femoral component with the radial arc centerline axis according to the method of any one of the first through third examples may also include aligning a medial condyle with the radial arc centerline axis. In a fifth example, the method of any one of the first through fourth examples may include selecting the tibial bearing insert prior to the identifying step, the tibial bearing insert being selected from among a plurality of tibial bearing inserts having one or more sizes and one or more articular surface profiles. In a sixth example, the method of any one of the first through fifth examples may include determining the initial articular surface of the tibial bearing insert prior to the identifying step by matching the initial articular surface with an anatomy of a patient intended to receive the tibial bearing insert. In a seventh example, the method of any one of the first through sixth examples may include performing each step of the method using a computer system. The computer system may include a processor, a memory and a storage device to store data. In an eighth example, a kit may include a plurality of tibial bearing inserts and a baseplate. The plurality of tibial bearing inserts may be designed in accordance with the method of any one of the first, second, and fourth through seventh examples. Among the plurality of tibial bearing inserts, at least two may have different sized outer perimeters. The baseplate may be adapted to receive each tibial bearing insert of the plurality of tibial bearing inserts. In a ninth example, the kit of the eighth example may be arranged such that each tibial bearing insert of the plurality of tibial bearing inserts includes a latch disposed therein, the latch being operable between unlocked and locked positions such that when one tibial bearing insert of the plurality of tibial bearing inserts is disposed on the baseplate, the one tibial bearing insert may be held in place with respect to the baseplate when the latch is in the locked position.

In yet another aspect, the present disclosure relates to a non-transitory, computer-readable medium storing one or more instructions. In a first example of a first embodiment, the non-transitory, computer-readable medium storing one or more instructions is configured such that when such instructions are executed by a computer system, the computer system is caused to: identify a radial arc centerline axis of a tibial bearing insert, the radial arc centerline axis passing through an apex of a lateral radial arc of the tibial bearing insert and an apex of a medial radial arc of the tibial bearing insert, both in a first transverse plane, the lateral and medial radial arcs representing lines of minimum thickness of the tibial bearing insert between an anterior side and a posterior side of the tibial bearing insert on an initial articular surface of the tibial bearing insert; align a center axis of a femoral flexion radius of a femoral component with the radial arc centerline axis such that the center axis and the radial arc centerline axis are coincident with a first coronal plane; while the center axis is maintained in the first coronal plane, align the femoral component with the tibial bearing insert by positioning the femoral component such that an inferior most point of a circle representative of the femoral flexion radius and centered on the center axis defines a predetermined minimum thickness of the tibial bearing insert; and subsequent to aligning the center axis and aligning the femoral component with the tibial bearing insert, modify a curvature of the initial articular surface of the tibial bearing insert between the anterior side and the posterior side to a modified articular surface that corresponds to a portion of the circle.

In a second example of the first embodiment, the medium of the first example may, when executed by a computer system, cause the computer to receive data representative of the tibial bearing insert and the femoral component prior to identifying the radial arc centerline axis.

### BRIEF DESCRIPTION OF THE DRAWINGS

A more complete appreciation of the subject matter of the present disclosure and of the various advantages thereof can be realized by reference to the following detailed description in which reference is made to the accompanying drawings in which:
FIG. 1 is a first perspective view of a tibial baseplate according to one embodiment of the disclosure;
FIG. 2 is a second perspective view of the tibial baseplate of FIG. 1;
FIG. 3 is a top-down view of the tibial baseplate of FIG. 1;
FIG. 4 is a cross-sectional side view of the tibial baseplate of FIG. 1;
FIG. 5 is a first perspective of a tibial insert according to one embodiment of the disclosure;
FIG. 6 is a second perspective view of the tibial insert of FIG. 5;
FIG. 7 is an anterior side view of the tibial insert of FIG. 5;
FIG. 8 is a cross-sectional side view of the tibial insert of FIG. 5;
FIG. 9 is a cross-sectional top view of the tibial insert of FIG. 5;
FIG. 10 is a perspective view of a latch according to one embodiment of the disclosure;
FIG. 11 is an anterior side view of a tibial implant system according to one embodiment of the disclosure;
FIG. 12 is a cross-sectional side view of the tibial implant system of FIG. 11;
FIG. 13 is a cross-sectional bottom-up view of the tibial implant system of FIG. 11 in an unlocked configuration;
FIG. 14 is a cross-sectional bottom-up view of the tibial implant system of FIG. 11 in a locked configuration;
FIG. 15 is a tibial implant kit according to one embodiment of the disclosure;
FIG. 16 is a perspective view of a tibial insert according to one embodiment of the disclosure;
FIG. 17 is a perspective view of a latch according to one embodiment of the disclosure;
FIGs. 18, 19 and 20 show steps in a method of locking a latch of a tibial implant system according to one embodiment of the disclosure; and
FIGs. 21 and 22 are steps in a method of designing a tibial insert according to one embodiment of the disclosure.

### DETAILED DESCRIPTION

As used herein unless stated otherwise, the term "proximal" means closer to the heart, and the term "distal" means further from the heart. The term "anterior" means toward the front part of the body or the face, the term "posterior" means toward the back of the body. The term "medial" means closer to or toward the midline of the body, and the term "lateral" means further from or away from the midline of the body. The term "inferior" means closer to or toward the feet, and the term "superior" means closer to or toward the crown of the head. As used herein, the terms "about," "approximately," "generally," and "substantially" are intended to mean that slight deviations from absolute are included within the scope of the term so modified.

In a first aspect, the present disclosure relates to a modular implant system that includes a bone anchorage component, an articular surface component attachable to the bone anchorage component and a fastener such as a latch to facilitate the attachment. This system may also be referred to as a universal insert-baseplate interface. In the embodiments described below, the system is used in a knee joint where the bone anchorage component is a tibial baseplate and the articular surface component is a tibial insert. However, it should be appreciated that the principles of the present disclosure may be applied to other hinge joints, such as the elbow and the ankle, and ball-and-socket joints, such as the hip and shoulder.

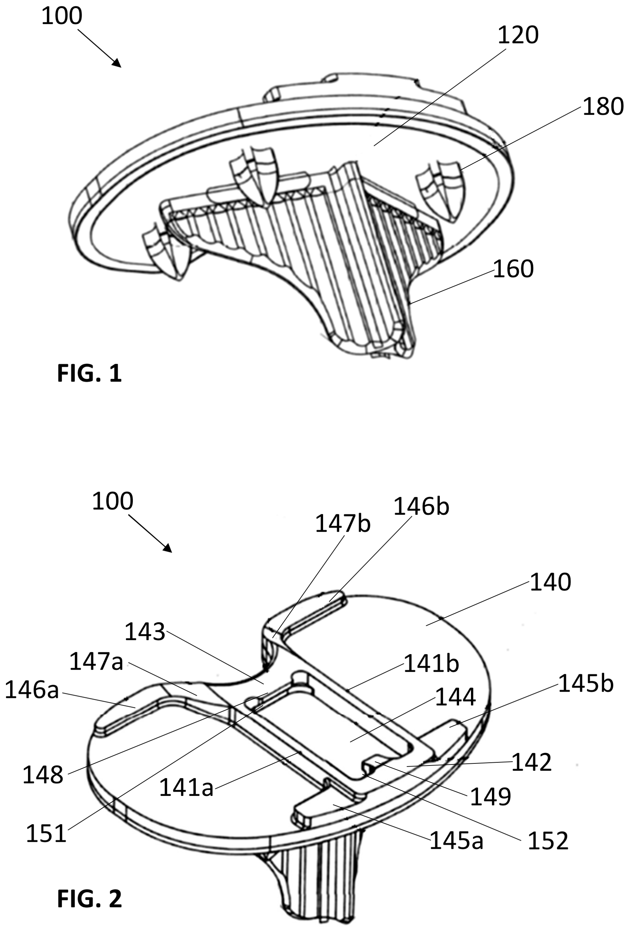

In one embodiment, a modular implant system in the form of a tibial implant system 50 may include a baseplate 100, a bearing insert 200 and a latch 260. As shown in FIG. 1, baseplate 100 includes a distal surface 120 for coupling with a tibial bone and a proximal surface 140 for coupling with a tibial insert. Distal surface 120 includes a keel 160 to resist torsional loads as well as translative loads in the anterior-posterior and medial-lateral directions. Additionally, distal surface 120 includes four fixation spikes 180 (three are visible in FIG. 1) to further resist relative movement between the baseplate and the bone under physiologic loads. Such function of the fixation spikes may be obtained where distal surface includes at least two fixation spikes, a variation that is also contemplated. Further, in some examples, a distal surface of the baseplate may have a single fixation spike. In other examples, the distal surface may include only one of a keel and one or more fixation spikes. In still further examples, the distal surface may include other known anchorage components other than a keel or fixation spikes.

As shown in FIGS. 2-4, baseplate 100 includes a proximal surface 140 opposite the distal surface. Proximal surface 140 includes an anterior wall 142 and a posterior wall 143. Anterior wall 142 includes a posterior-facing protrusion 149 that defines an anterior recess 152 thereunder. Posterior wall 143 includes an anterior-facing protrusion 148 that defines a posterior recess 151 thereunder. In the depicted embodiment as shown in FIG. 2, proximal surface 140 includes a first rail 141A oriented in the sagittal plane offset from the midplane of the baseplate in a medial direction and a second rail 141B oriented in the sagittal plane offset from the sagittal midplane of the baseplate in a lateral direction, said first and second rails being substantially parallel. Each of the first and second rails extends between the anterior wall and the posterior wall. Additionally, anterior wall 142 connects first and second rails 141A, 141B while posterior wall 143 also connects first and second rails 141A, 141B. In these arrangements, a recessed pocket 144 is formed between first and second rails and anterior and posterior walls.

With continued reference to FIG. 2, proximal surface 140 also includes a first anterior abutment 145A connected to first rail 141A and a second anterior abutment 145B connected to second rail 141B. In some examples of this embodiment, including that shown in FIG. 2, at least one wall of the first and second anterior abutments 145A, 145B may be substantially perpendicular to first and second rails 141A, 141B. Similarly, proximal surface 140 also includes a first posterior abutment 146A and a second posterior abutment 146B. First posterior abutment 146A is connected to first rail 141A by a first angled wall 147A and second posterior abutment 146B is connected to second rail 141B by a second angled wall 147B whereby the included angle between first angled wall 147A and second angled wall 147B is approximately 30 degrees. In variations, the angle between the first angled wall and the second angled wall may be any value from approximately 15 degrees to approximately 45 degrees.

In some variations, at least one surface of the first posterior abutment 146A is substantially parallel to the first anterior abutment 145A and at least one surface of the second posterior abutment 146B is substantially parallel to the second anterior abutment 145B. In other examples of the above-described embodiment, a proximal surface of the baseplate includes an anterior wall and a posterior wall as described above, while optionally including one or more of the first rail, second rail, first anterior abutment, second anterior abutment, first posterior abutment, second posterior abutment, first angled wall and second angled wall.

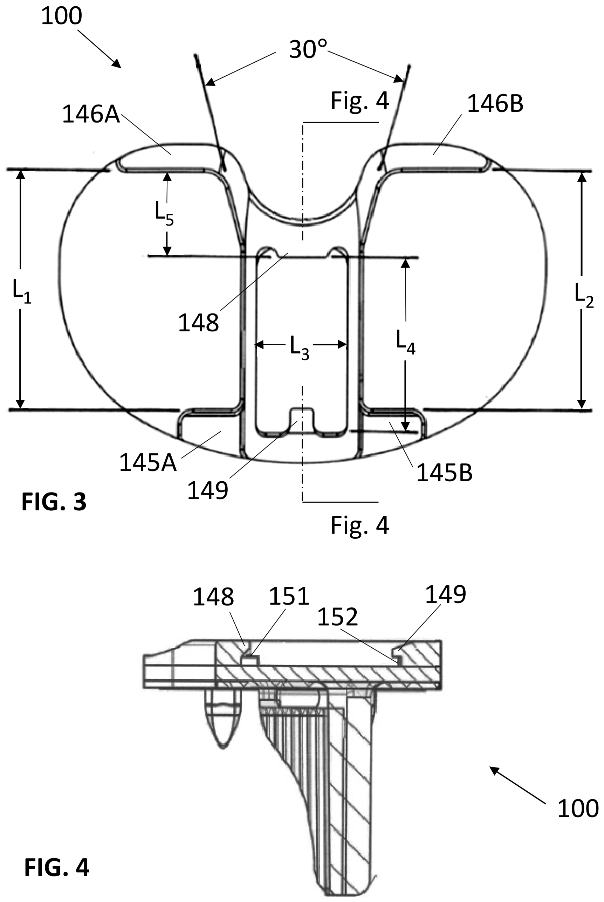

While FIGs. 1-4 show a single baseplate 100, it should be appreciated that the present disclosure contemplates multiple baseplate sizes compatible with a single tibial insert and vice versa, as is described in greater detail below. This compatibility is facilitated in part through the consistent incorporation of dimensions L1, L2, L3, L4 and L5 throughout a contemplated range of baseplate sizes, such dimensions being indicated in FIG. 3. Specifically, the distance L1, L2 between the posterior and anterior abutments on respective medial and lateral sides of the baseplate is constant for two or more sizes of the baseplate, as is the width L3 and length L4 of the recessed pocket and its dimensional relationship L5 to the anterior and posterior abutments of the baseplates.

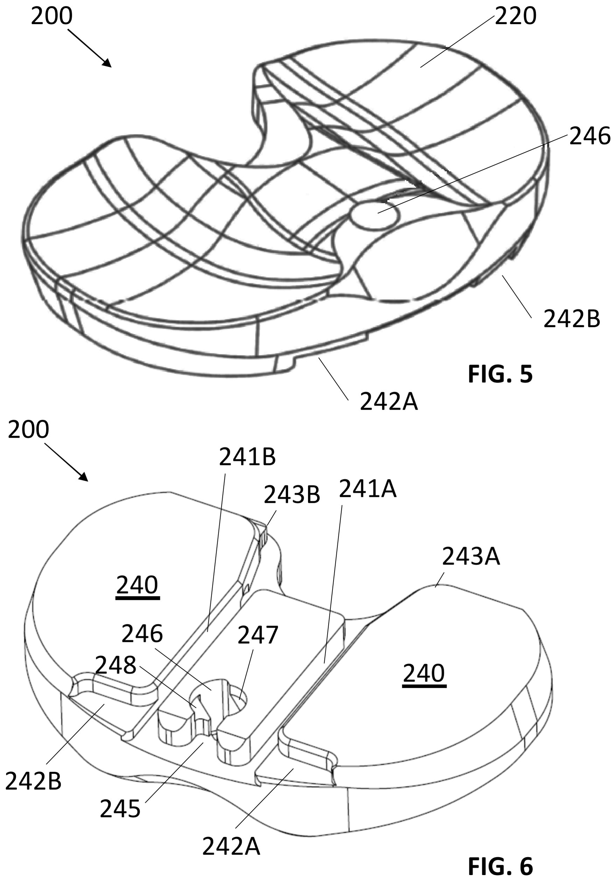

Bearing insert 200 of tibial implant system 50 is shown in FIGS. 5-9 and includes a proximal surface 220 for interfacing with a femoral knee component as an articular surface and a distal surface 240 for interfacing with the proximal surface of the tibial baseplate, *e.g.*, baseplate 100. As shown in FIG. 6, distal surface 240 includes a first groove 241A and a second groove 241B for coupling with the first rail 141A and second rail 141B of the baseplate, respectively. The first and second grooves are dimensioned to cause an interference fit upon assembly. Distal surface 240 further includes a first anterior recess 242A and a second anterior recess 242B to couple with first abutment 145A and second abutment 145B respectively and a first posterior recess 243A and a second posterior recess 243B to couple with first 146A and second 146B posterior abutments of the baseplate. The first and second anterior and posterior recesses and the first and second anterior and posterior abutments are dimensioned to cause an interference fit upon assembly. All interference fits herein described resist torsional and translational movement of the insert with respect to the baseplate when subjected to physiological loads.

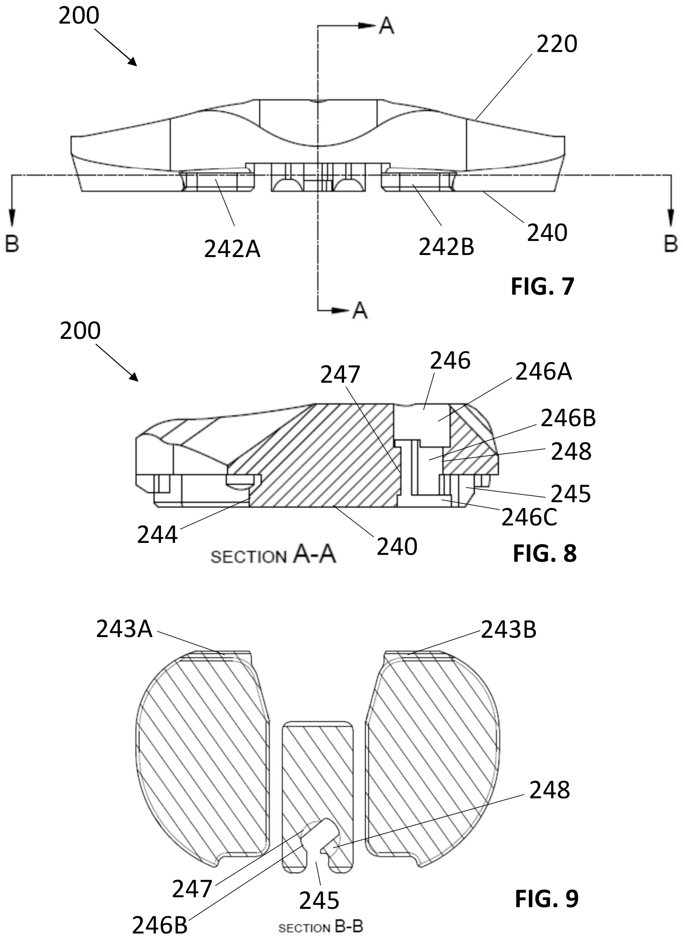

As shown in FIG. 8, distal surface 240 of bearing insert 200 includes features arranged for interfacing with a baseplate. In particular, distal surface 240 includes a posterior protrusion 244 disposed between first groove 241A and second groove 241B for coupling with posterior recess 151 of baseplate 100 to prevent posterior lift-off of the insert from the baseplate. Further, and as shown in FIGS. 6, 8 and 9, distal surface 240 of the bearing insert includes an anterior recess 245 disposed between first groove 241A and second groove 241B for coupling with baseplate protrusion 149 of baseplate 100. In some alternative examples of the bearing insert, the distal surface of the bearing insert may include the protrusion and recess to engage with respective protrusions and recesses in the posterior and anterior walls of the baseplate, while optionally including one or more of first groove, second groove, first anterior recess, second anterior recess, first posterior recess and second posterior recess.

With reference to FIGS. 5, 6 and 8, bearing insert 200 also includes a multi-stepped hole 246 therethrough, the hole being perpendicular to distal surface 240 and having a length from the distal surface to proximal surface 220. Further, and as shown in FIG. 8, multi-stepped hole 246 has a varying cross-sectional shape along its length. Beginning at distal surface 240, hole 246 is defined by lower wall 246C. Lower wall 246C includes a concave wall surface portion and an anterior-facing open portion leading to anterior recess 245. Adjacent to the lower wall in a direction toward the proximal surface, hole 246 becomes narrower in a central wall 246B and is defined in part by rear wall projection 247 and forward wall projection 248. Central wall 246B also includes concave portions over at least some distance in between the wall projections. In the depicted embodiment, these wall projections each have a generally flat surface and are parallel and opposite to one another, as shown in FIGS. 6 and 9. Each wall projection 247, 248 is entirely interior to surfaces 220, 240 such that regions of hole 246 adjacent to such surfaces 220, 240 are spaced apart from the wall projections. Adjacent to the central wall in a direction toward the proximal surface is an upper wall 246A of hole 246. The upper wall portion is wider than the central wall portion. Upper wall 246A of the hole abuts proximal surface 220 and includes a generally concave surface that defines its shape. In some examples, including that depicted in FIGs. 5-9, the surface of upper wall 246A may be circular in cross-section.

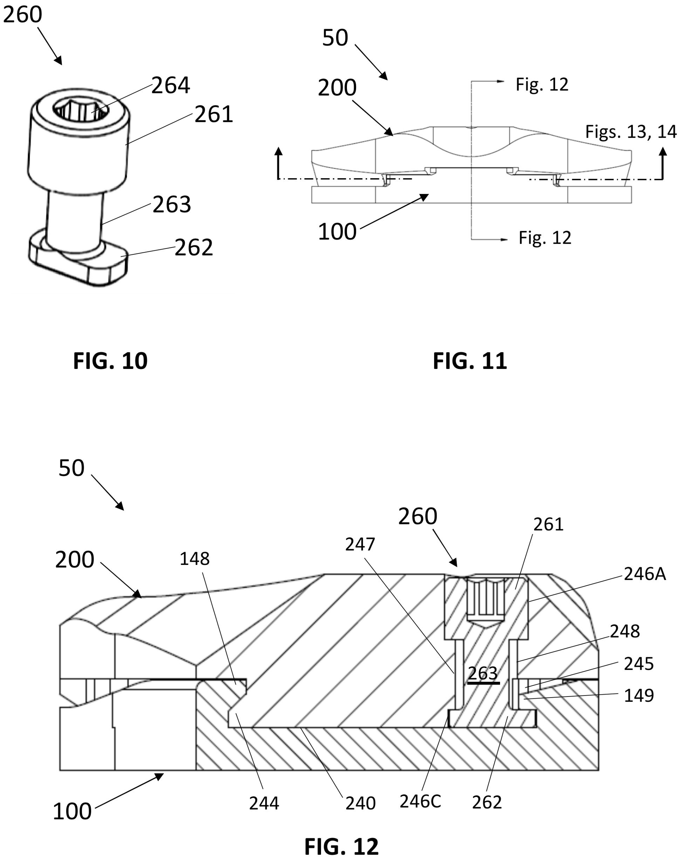

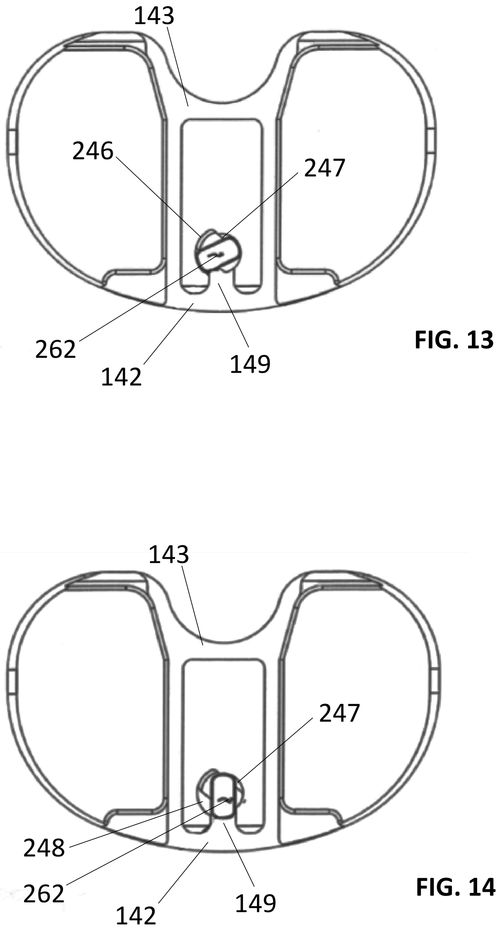

In some embodiments, tibial implant system 50 may also include a fastener adapted to hold the bearing insert in place with respect to the baseplate. One example of a fastener is shown in FIG. 10 in the form of a latch 260. Latch is sized for disposal through multi-stepped hole 246 and for manipulation therein. The latch may be factory assembled with the bearing insert such that the latch is disposed within the bearing insert prior to use, and may be further configured to prevent disassembly once initially assembled. Upon assembly, the latch is operable between unlocked and locked positions. The unlocked and locked positions are shown in FIGS. 13 and 14 respectively and are described in greater detail elsewhere in the present disclosure as part of a method of using the system. Latch 260 includes an outer shaft portion 261, a central shaft portion 263 and a tab portion 262. Central shaft portion 263 is in between the other portions, as shown in FIG. 10. Within at least part of outer shaft portion 261 is a drive aperture 264. Aperture 264 may be a hex for engagement by a wrench to manipulate latch 260. As shown in FIG. 10, outer shaft portion 261 and central shaft portion 263 may be cylindrical in shape. Outer shaft portion 261 has a larger diameter than central shaft portion 263. Central shaft portion 263 has a length, measured in axis passing through the different portions of the latch, that is similar to or longer than a length of central wall 246B of hole 246. Tab portion 262 may have an elongate shape such that a long dimension of tab portion 262 is greater than a diameter of central shaft portion 263. In some examples, the long dimension of tab portion 262 may be greater than a diameter of outer shaft portion 261. The tab portion may include sidewalls 262A, 262B extending along the long dimension. In some examples, the sidewalls may have a generally linear alignment. A thickness of tab portion 262 may be less than a depth of lower wall 246C in hole 246. In some examples, the sidewalls may have a flat surface and may be parallel to one another, as is shown in FIG. 10. The tab portion may have beveled edges to reduce the risk of damage to the system components during use.

As to the size of latch 260, the latch may be sized to file within hole 246 such that there is a friction fit when the latch is disposed in the hole. In this manner, the latch will not rotate or otherwise move relative to the bearing insert unless an active force is applied, such as through engagement and rotation of aperture 264 with a drive tool.

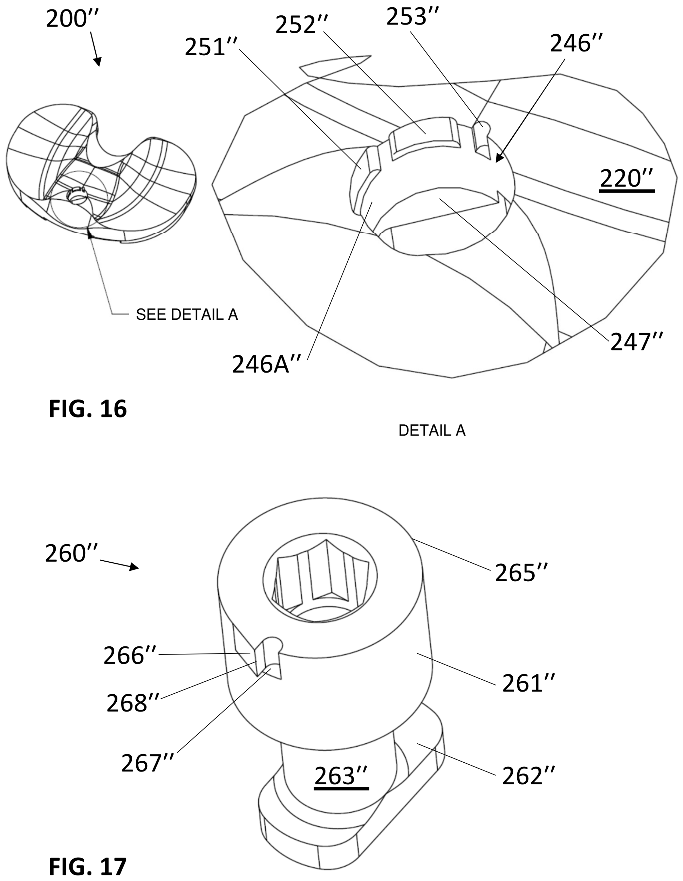

In some embodiments, the modular implant system may include a bearing insert 200" and latch 260" as shown in FIGs. 16 and 17, respectively. Reference numerals indicated by the 200" double-prime series of numerals refer to like reference numerals in the 200 series of reference numerals, unless otherwise indicated. In these embodiments, bearing insert 200" includes a hole 246" for receipt of latch 260". Located on upper wall 246A" of the hole are three separate indentations, each on an outside edge of the hole abutting proximal surface 220". Assembly indentation 251" extends circumferentially along a periphery of hole 246" over a first length, first operative indentation 252" extends circumferentially along the periphery of the hole over a second length, and second operative indentation 253" extends along the periphery of the hole over a third length. In some examples, and as depicted, the first length is greater than the second length and the second length is greater than the third length. Each of the aforementioned indentations may be arranged in sequence such that the first operative indentation is in between the assembly indentation and the second operative indentation. An elongate surface of assembly indentation 251" and first operative indentation 252" may be generally parallel to upper wall 246A". Second operative indentation may be defined by a rounded surface such that its circumferential length is less than its depth along a length of the hole. In between the first and second indentations is first interruption 258" and in between second and third indentations is second interruption 259". Each of these interruptions may have a concave surface that is generally complementary to the convex surface of the latch.

Latch 260" includes outer shaft portion 261" with an outer trailing edge 265" defining a periphery of the latch at its trailing end. Included on the outer shaft portion abutting the outer trailing edge are projection 266" and indentation 267", each extending a distance generally parallel to a longitudinal axis of the latch from outer trailing edge 265". Projection 266" may have a flat surface extending from a circumferential surface of outer shaft portion 261" that flares outward relative to the outer shaft portion toward a projection outer edge 268". In some examples, and as depicted in FIG. 17, indentation 267" may immediately abut projection outer edge 268". Further, in some examples, a surface of indentation 267" facing projection 266" may be flat and perpendicular to the axis of rotation of the latch. In the above examples, projection outer edge 268" represents a latch location that is a maximum radial distance from a central longitudinal axis of the latch, where part of the flat surface of the indentation is exterior to the convex outer surface of outer shaft portion 261". In some examples, projection 266" may be integrally formed, e.g., machined, with a remainder of the latch.

Latch 260" and hole 246" may be sized such that there is an interference fit between the components. The interference fit may be limited to the extent that rotational movement of the latch remains possible while the latch is disposed in the hole. For example, the interference fit may prevent unintended rotation of the latch such that the latch will not rotate under passive conditions. Projection 266 may provide a more significant interference fit than a remainder of the outer surface of the latch. In some examples, the insert and the latch may be formed through a machining process to optimize the interference fit. Assembly indentation 251" and first operative indentation 252" provide clearance relief for the projection 266" to aid in facilitating clockwise rotation of the latch within the insert. Interruptions 258", 259" may prevent counterclockwise rotation of the latch when projection 266" is disposed clockwise relative to either interruption. Ease of clockwise rotation of the latch may be adjusted based on a length the circumferential indentations on the insert. For example, a shorter first operative indentation 252" will increase the force necessary to rotate the latch in a clockwise direction and reduce the likelihood of accidental clockwise rotation. Further detail regarding the operative relationship between the latch and the bearing insert is described elsewhere in the present application.

Components of the system may be made of a variety of materials. In some examples, baseplate 100 may be made of a metal such as titanium. In other examples, baseplate may be made of a polymeric material such as polyether either ketone ("PEEK"). In still further examples, the baseplate may be made of a polymer-metal composite. In some examples, bearing insert 200 may be made of a polymeric material such as ultra-high molecular weight polyethylene. In further examples, one or both of the baseplate and insert may be made of ceramic materials.

The modular implant system may be varied in many ways. For example, the bearing insert and baseplate may be shaped so that the posterior protrusion on the bearing insert is anterior-facing and the posterior-side protrusion on the baseplate is a hook that is posterior-facing. In such a configuration, the bearing insert may be passed around the posterior end of the baseplate and then brought back toward the baseplate to engage the components on the posterior side, from which position the posterior-side of the bearing insert is prevented from lift-off by the hook of the baseplate. In another example, the fastener may be a rod with movable parts that are mechanically actuated, in place of a latch. In such arrangements, the rod may have an unlocked position where the rod freely slides through a hole in the bearing insert, and a locked position where a lateral projection extends from the rod to prevent the rod, and the bearing insert, from being removed from the baseplate. The lateral projection may be controlled from a superior-facing end of the rod that is exposed even when the rod is disposed in the bearing insert. In one specific example, pulling or pushing may control the extent of the lateral projection to move the lateral projection between the unlocked and locked positions. In variations of this example, such rod may be monolithic with the bearing insert.

In other embodiments, a system may include any combination of one or more of a baseplate, bearing insert and fastener, such as a latch, as described above, where each component may have features based on the variations of such components as contemplated by the present disclosure.

In another aspect, the present disclosure relates to a kit with components for a modular implant system. In one embodiment, a kit includes a latch, at least one baseplate and at least one bearing insert. The kit of this embodiment may include any number of baseplates and any number of bearing inserts. Two or more of the baseplates may have different sizes and two or more of the bearing inserts may have different sizes. A size of the baseplate or bearing insert may be its footprint in terms of its surface area facing a joint for which it forms a part. When the components are for the knee, this may be a surface area in a transverse plane over a proximal end of the tibia. Where there are baseplates of different sizes, each size of baseplate will have the same L1, L2, L3, L4 and L5 dimensions as indicated in FIG. 3. These singular dimensions may themselves vary from kit to kit, but all baseplates in the kit will be sized to have matching dimensions in the above respect. Similarly, the bearing inserts, whether or not they are provided in one or more sizes, will have distal surface contours with dimensions that match L1, L2, L3, L4 and L5, or a subset thereof for certain baseplate designs as applicable, such that any one of the bearing inserts is attachable to any one of the baseplates. In examples where one or more of the rails and abutments are absent from the baseplates, the baseplates of the kit will share dimension L4 but may or may not include the remaining shared features. In such examples, the set of bearing inserts will have shared dimensions to the same extent as those found in the baseplates. Thus, where baseplates of a kit only share dimensions L3 and L4, the bearing inserts of the kit may also only share dimensions L3 and L4.

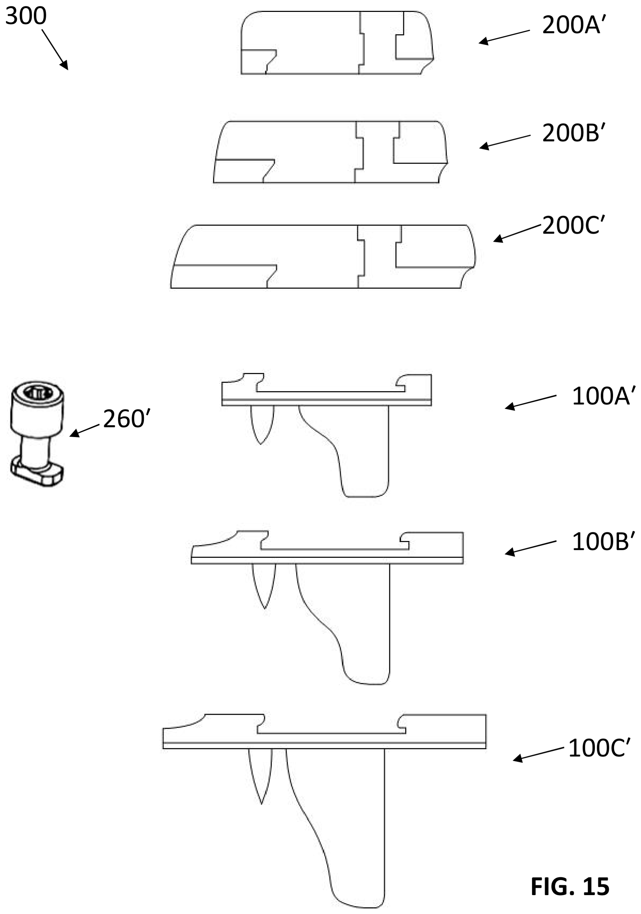

In one specific example, a kit 300 includes a latch 260', three baseplates 100A', 100B', 100C' each having different sizes and three bearing inserts 200A', 200B', 200C' each having different sizes, as shown in FIG. 15. In FIG. 15, reference numerals indicated by prime ("'") refer to like reference numerals without prime, such that a reference to 100' refers to like reference numeral 100. For each of the three baseplates, the dimensions indicated by L1-L5 in FIG. 3 are the same, and each of the three bearing inserts is compatible with any one of the three baseplates. In this manner, any one of the bearing inserts may be attached to the smallest baseplate, the medium sized baseplate or the largest baseplate. And, the smallest bearing insert, the medium sized bearing insert or the largest bearing insert may be attached to any one of the baseplates. In another example, at least one of the one or more baseplates is baseplate 100. In yet another example, at least one of the one or more bearing inserts is insert 200. In this embodiment and in all of the illustrative examples, the latch is compatible with any combination of baseplate and bearing insert and may be used to lock and unlock the two components. Thus, latch 260' is receivable in any one of bearing insert 200A', 200B', 200C' and when one of such bearing inserts is attached to one of baseplates 100A', 100B', 100C', latch 260' may be actuated between an unlocked and a locked position.

In another embodiment, a kit may include a latch, at least one baseplate, at least one bearing insert and a tool for actuating the latch, such as a driver tool. In yet another embodiment, a kit may include at least one baseplate and at least one bearing insert. In some examples of this embodiment, the kit may include two or more baseplates having different sizes. In still further examples of this embodiment, the kit may include two or more bearing inserts having different sizes. In still further embodiments, a kit may include a latch and one or more bearing inserts. In other embodiments, a kit may include a latch and one or more baseplates.

In another aspect, the present disclosure relates to a method of manufacturing one or more components of a modular implant system, such as a tibial implant system. In one embodiment, one or more of the baseplate, bearing insert and latch are formed using additive manufacturing methods. In some examples, additive manufacture may be layer-by-layer using an additive layer manufacturing ("ALM"), *i.e.,* 3D printing, process. With formation of the implant components using ALM, the need for assembly of multiple components is, in many instances, eliminated. In some examples, ALM processes are powder-bed based and involve one or more of selective laser sintering ("SLS"), selective laser melting ("SLM"), and electron beam melting ("EBM"), as disclosed in U.S. Pat. Nos. 7,537,664; 8,728,387; 9,180,010; and 9,456,901, the disclosures of which are hereby incorporated by reference herein in their entireties. ALM processes may also be performed using fused deposition modeling ("FDM") or other appropriate 3D printing technologies known to persons skilled in the art. When employing powder-bed based technologies, articles are produced in layer-wise fashion according to a predetermined digital model of such articles by heating, e.g., using a laser or an electron beam, multiple layers of powder, which may be a metallic powder, that are dispensed one layer at a time. The powder is sintered in the case of SLS technology and melted in the case of SLM technology, by the application of laser energy that is directed in raster-scan fashion to portions of the powder layer corresponding to a cross section of the article. After the sintering or melting of the powder on one particular layer, an additional layer of powder is dispensed, and the process repeated, with sintering or melting taking place between the current layer and the previously laid layers until the article is complete. The powder layers similarly may be heated with EBM technology. It should also be appreciated that other devices, instruments, or components therefor that are contemplated by this disclosure may also be formed through an ALM process. In some instances, materials for one layer may be different than the materials for successive layers.

In some embodiments, through an ALM process or otherwise, the latch of the system may be formed together with the bearing insert. In this manner, once the bearing insert and latch are formed, the latch is held in place within the bearing insert. In some variations, the latch may be sized and shaped such that the latch is irremovable from the bearing insert once assembled therein, while still remaining rotatable to control whether the bearing insert is locked to the baseplate when the bearing insert is disposed on the baseplate. In other examples, the latch may be removably held within the baseplate. In this configuration, the latch may be removed entirely from the bearing insert, or reinserted after removal, provided that the latch is in the unlocked position during those actions.

In another aspect, the present disclosure relates to a method of assembling a modular implant system for a surgery. Use of the method allows for the assembly of a tailored articular surface size, e.g., bearing insert, and a tailored implant for bone engagement, e.g., baseplate. Included among the contemplated methods is assembly of a tibial implant system for a knee surgery, where the baseplate is sized for the proximal tibia and the bearing insert is sized for optimal interaction with the femoral condyles. In one embodiment, a method involves obtaining bearing insert 200 with latch 260 pre-assembled therein and a baseplate. A choice may be made at this step as to what size of bearing insert and baseplate should be combined into an assembly. In one illustration, where available, a bearing insert size may be chosen for compatibility with a distal femoral implant, e.g., articular surface of femoral condyles, and a baseplate may be chosen for compatibility with a proximal end of a tibial bone of a patient.

With the bearing insert and baseplate chosen, bearing insert 200 is slid onto baseplate 100 by sliding posterior protrusion 244 of the bearing insert into posterior recess 151 of the baseplate. During this step, if the bearing insert includes first and second grooves and the baseplate includes anterior and posterior abutments, as is shown in FIGs. 6 and 2, respectively, then the body of the bearing insert may be restrained against translation in the posterior direction by the posterior abutments and against translation in the anterior direction by the anterior abutments, while the first and second grooves of the bearing insert are received in the first and second rails of the baseplate to restrain translation in the medial-lateral direction. At the time the bearing insert arrives in position on the baseplate via engagement between posterior protrusion 244 and recess 151, as shown in FIG. 12, latch 260 is initially in the unlocked position. While the latch is in the unlocked position, as shown in FIG. 13, the bearing insert may still be susceptible to vertical displacement relative to the baseplate, i.e., in a superior direction if the baseplate were in place on a tibia, particularly on the anterior side. To complete assembly, latch 260 may be actuated from the unlocked position to the locked position. In system 50 as shown, this involves actuating drive 264 of latch to rotate latch from the unlocked position shown in FIG. 13 to the locked position shown in FIG. 14, thereby engaging the baseplate by bringing tab portion 262 of the latch into anterior recess 152 under posterior-facing protrusion 149, as shown in FIG. 12. In this manner, protrusion 149 now prevents anterior lift-off of bearing insert 200 from baseplate 100.

In a variation of the above embodiment, the method of assembly further comprises an initial step of sliding the latch into place within the bearing insert. The latch is first oriented in the unlocked position to pass into and through the central wall portion of hole 246 in order to fully dispose the latch into the bearing insert. The method may otherwise proceed as already described from this step onward. In other examples, the method may be employed in reverse to remove a bearing insert from a baseplate by rotating the latch clockwise from the locked position to the unlocked position.

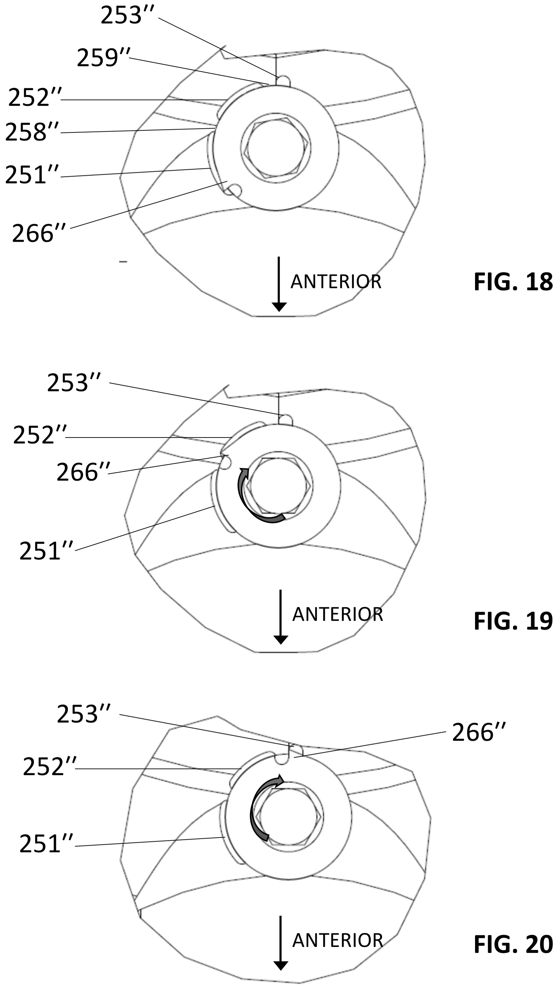

In some embodiments, the method of assembling a modular implant system may involve the use of bearing insert 200" and latch 260" along with a baseplate. The baseplate may be a baseplate as contemplated in embodiments of the present disclosure, such as baseplate 100. In some examples, the method may begin with the latch predisposed in the bearing insert and oriented as shown in FIG. 18, for example. In this latch position, outward protrusion 266" is within assembly indentation 251". In other examples, the method may include an initial preceding step of inserting the latch into the bearing insert by orienting the latch into a position such as that shown in FIG. 18 and then advancing the latch into the hole. This insertion step involves aligning tab 262" with sidewalls 247, 248 of the hole (see FIG. 9 for example). Preserving such orientation allows for the latch to be fit into the hole of the bearing insert.

After the latch is positioned in an orientation as shown in FIG. 18, also referred to as an assembly orientation, the latch is rotated into a position as shown in FIG. 19 so that outward protrusion 266" of the latch is disposed in first operative indentation 252". This latch position may also be referred to as an unlocked position and is a rotational position of the latch when ready for matching the insert with a baseplate during use in a surgical procedure. In the unlocked position, the latch is held in place within the insert and will remain within the insert even without being held in place. A baseplate is then identified for use with bearing insert 200", and once bearing insert is fit onto the baseplate as described elsewhere in the present disclosure, latch 260" is rotated further to bring protrusion 266" into second operative indentation 253", as shown in FIG. 20. This latch position may also be referred to as a locked position. In the locked position, tab portion 262" of the latch is held under a posterior-facing protrusion of the baseplate so that the bearing insert is held in place with respect to the baseplate.

During operation of the latch to rotate the latch from the assembly position to the unlocked position, or to rotate the latch from the unlocked position to the locked position, projection 266" encounters significant resistance to pass over first and second interruptions 258", 259". When the insert is made of polymeric materials, such as UHMWPE, a surface of the insert defining hole 246" may stretch to provide sufficient clearance for the latch to rotate in the clockwise direction to the desired position. Once projection 266" rotates past the interruption, hoop stresses and creep in the insert material lead to the insert returning to its shape extant prior to the change in rotational position of the latch. In this way, potential counterclockwise rotation is prevented once the projection 266" passes an interruption.

In variations of the above embodiments, the method may commence with the latch in the unlocked position within the bearing insert and continue from that step. In still further variations of the method, the bearing insert may be removed from the baseplate by further clockwise rotation of the latch from the locked position in FIG. 20 to the assembly position or the unlocked position. Such further rotation may require significantly greater torque to rotate the latch when the hole surface between indentation 253" and indentation 251" does not include additional indentations, as is the case for the embodiment shown in FIGs. 16-20. In further variations where the hole includes additional indentations for such purpose of resetting the rotational position of the latch, such rotation may require lesser torque.

In yet another aspect, the present disclosure relates to a method of using a modular implant system. In one embodiment, the method commences with retrieval of the implant components of the system. In some examples, the implant components are already assembled. In other examples, the method may include an initial step of assembling the components. Such assembly may be performed as contemplated by the methods of the present disclosure. Then, known methods may be utilized to prepare a patient, create access at a surgical site, and implant the system, such as tibial implant system, in the patient. In a variation, the method may also include an initial step of choosing a bearing insert and baseplate size, followed by assembly of the bearing insert and baseplate and the performance of surgery.

In another aspect, the present disclosure relates to a method of designing a bearing insert to optimize medial congruency between the bearing insert and an implant in operative contact with an articular surface of the bearing insert. In the embodiments described below, the method is described for knee implant components where the articular surface component is a tibial bearing insert and the component in operative contact with the articular surface is a distal femoral component. However, it should be appreciated that the principles of the present disclosure may be applied to other hinge joints, such as the elbow and the ankle, and ball-and-socket joints, such as the hip and shoulder. Further, it should also be appreciated that the steps of the method of this aspect may be performed using a computer system. Such computer system may include software for generating images or data for the implant components of the joint, along with any coordinates, lines, axes and other pertinent identifiers for locations in space. The images or data may be displayed on a display for analysis and consideration by a user of the system. Similarly, the computer system may also be equipped with a user interface to allow for user input to facilitate the design process.

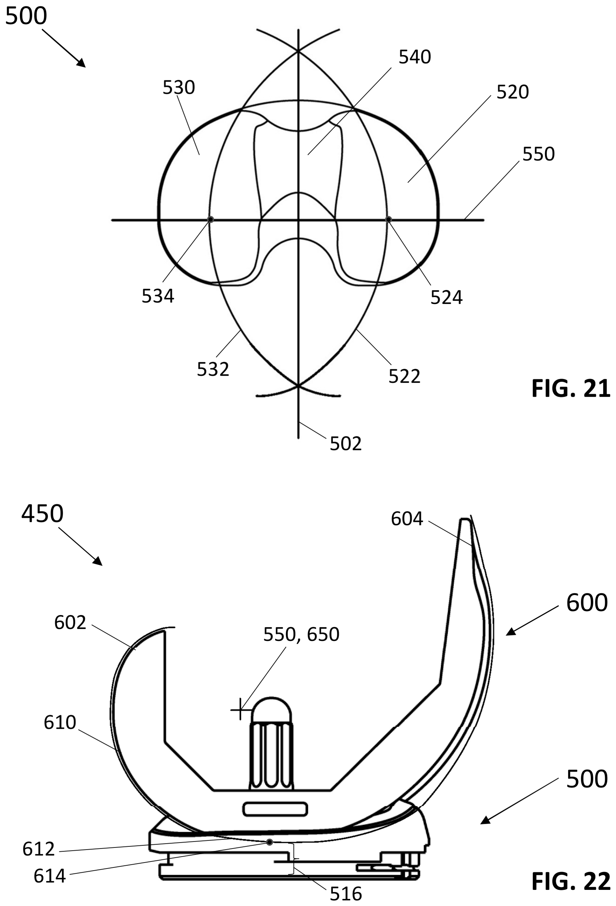

In one embodiment, a method of design begins with selection of a bearing insert, such as bearing insert 500 shown in FIG. 21. Although the bearing insert in FIGS. 21 and 22 is for a left knee, it should of course be understood that the same principles as described for this method and indeed throughout the disclosure may also be applied to a right knee. Additionally, while the present embodiment is described with the inclusion of a bearing insert, it should be appreciated that the method may also involve a tibial component that includes both a bearing insert and a baseplate, either monolithically formed or as attachable components, such as those described elsewhere in the present disclosure. Bearing insert 500 includes a medial articular surface 520 and a lateral articular surface 530. Each of these articular surfaces includes a radial arc, also referred to as a radial track. Such radial arc is a low line of a trough on the concave surface on the medial and lateral sides, the low line extending along the surface between anterior and posterior sides of the insert in a generally anterior-posterior direction. Further details of bearing inserts with exemplary radial arcs are described in U.S. Patent No. 7,160,330, hereby incorporated by reference herein in its entirety. The bearing insert may be selected as appropriate for the surgery at issue. In this manner, the bearing insert may be chosen based on the tibial implant size needed, based on the femoral component size needed, or based on a combination of both. Where a kit such as kit 300 is utilized, the bearing insert may simply be chosen based on the femoral component size, since the bearing insert may be attached to any size baseplate for the tibial implant. It should be appreciated that the radial arc on the articular surfaces of the bearing insert may, in some examples, vary based on a size of the bearing insert. In other examples, the radial arcs of each bearing insert may be the same irrespective of the bearing insert size. In other circumstances, different bearing inserts having the same size may have varying radial arcs. In still further examples, a kit or set of bearing inserts may include bearing inserts that collectively have any combination of the foregoing characteristics. These examples are merely to illustrate that the radial arc on a given bearing insert need not be limited to particular shape nor need it be conditioned on having certain detailed parameters in order to proceed with the contemplated method of design.

With a bearing insert 500 selected, radial arcs 522, 532 of such bearing insert are identified in a transverse plane, as shown in FIG. 21. A medial-lateral centerline 502 of the bearing insert is also identified, such centerline passing anteriorly-posteriorly through a center of an intercondylar eminence 540 in the depicted embodiment. Each arc is then evaluated against the medial-lateral centerline to identify an apex or maximum distance from such centerline 502 along a portion of the arc on the articular surface. This is done for both the medial radial arc 522 and lateral radial arc 532, thereby identifying medial apex 524 and lateral apex 534. It should be appreciated that these apices are based on the curvature of the radial arcs in a transverse plane, and that accordingly such apices do not necessarily coincide with a low-point on the respective medial and lateral articular surfaces of the insert, i.e., minimum thicknesses in respective medial and lateral regions of the insert. However, because the radial arcs represent a low line or depression of an articular surface along a curved path between anterior and posterior sides of the insert, such radial arcs may represent a localized minimum thickness of the bearing insert in a mid-coronal plane through the insert and in other coronal planes anterior or posterior to the mid-coronal plane. With continued reference to FIG. 21, a line or axis passing through each of apices 524, 534 is a radial arc centerline axis 550. For the bearing insert in FIG. 21, the radial arc centerline axis is generally oriented medial-laterally.

In a subsequent step, shown in FIG. 22, femoral component 600 is retrieved. The femoral component size and characteristics may be selected based on a fit with a patient's distal femur at the knee joint. Other factors may also be considered for femoral component selection. For example, patient-specific kinematics. With femoral component 600 selected, femoral component is then viewed in a sagittal plane to align femoral component 600 with bearing insert 500. Together, femoral component 600 and bearing insert 500 may form a knee prosthesis 450. To align femoral component 600, a flexion radius center axis 650 is first identified. The flexion radius center axis represents a center of a portion of a condylar surface curve 612 of the femoral component, typically capturing a single center for both medial and lateral condylar surfaces. For the sake of brevity, further discussion focuses on medial condylar surface 610, though it should be appreciated that these steps may vary for patients with different natural kinematics. A portion of condylar surface 610 utilized for this determination may vary, but is focused on a region that is in contact with the bearing surface through a core range of motion in the joint. Thus, for example, flexion radius center axis 650 may be a center of a curve 612 matching a portion of the condylar surface in contact with the articular surface of the insert from approximately 10 degrees of flexion to approximately 110 degrees of flexion. For comparison, the femoral component 600 and bearing insert 500 as shown in FIG. 22 are at 0 degrees of flexion, and it is a portion of condylar surface 610 moving from the low point in FIG. 22 toward posterior side 602 of femoral component 600 that constitutes greater angles of flexion in the joint. In some examples, the range of the condylar surface used to determine the flexion radius may be different from the example above. For instance, the range may include condylar surfaces in contact with the insert from 0 degrees to 120 degrees of flexion. In other examples, the range may be from 30 degrees to 90 degrees of flexion. In still further examples, the range may be any subset of the aforementioned ranges, such as between 0 degrees and 60 degrees of flexion.

With flexion radius center axis 650 established relative to the femoral component position, the flexion radius center axis is then aligned with radial arc centerline 550 so that both axes are in a single coronal plane, i.e., in a plane at a set distance between anterior and posterior sides of the insert. To finalize a position of femoral component 600 relative to bearing insert 500, flexion radius center axis 650 must be aligned relative to bearing insert 500. To perform such alignment, a desired and/or minimally sufficient bearing insert thickness is confirmed, and a low point 614, i.e., inferior-most point, of condylar surface curve 612 is moved inferiorly or superiorly so that a thickness 516 of the bearing insert below the low point meets the confirmed thickness value. In this manner, flexion radius center axis 650, already aligned with radial arc centerline 550 in a coronal plane, is shifted in either a superior direction or in an inferior direction so that low point 614 is positioned to produce a bearing insert having the sought-after thickness. FIG. 22 shows femoral component 600 adjusted to account for both radial arc centerline 550 and a thickness of bearing insert 500. In one example, applicable law may dictate what constitutes a sufficient minimum bearing insert thickness. In other examples, performance characteristics of the bearing insert may be a controlling factor. In one specific example, a determined minimum thickness of the bearing insert may be approximately 6mm. In other examples, the minimum thickness of the bearing insert may be any value from 5mm to 10mm.

As shown in FIG. 22, the alignment of femoral component 600 with respect to bearing insert 500 indicates a difference between condylar surface curve 612, corresponding to a portion of the femoral component in contact with the bearing insert over a range of flexion in the joint, and an already established articular surface contour of bearing insert 500. This discrepancy is addressed by modifying a contour of medial articular surface 520 in a sagittal plane so that the articular surface of the insert conforms to condylar surface 610 via condylar surface curve 612. Put another way, the medial articular surface of the bearing insert is modified to match condylar surface curve 612 between anterior and posterior ends of the bearing insert. It should be appreciated that this modification is intended to update the design for an improved fit between a medial condyle and a medial articular surface of the insert, and thus may also include modifications of the articular surface curvature outside of the sagittal plane based on a shape of a medial condyle to the extent such modifications improve the fit.

Through this design process, a shape of medial articular surface 520 better conforms to the medial condyle of femoral component 600 so that the medial condyle is better held in place within medial articular surface 520 while the tibia of the patient moves relative to the femur through a range of motion. In this manner, the medial condyle of the femoral component and the medial articular surface of the bearing insert are shaped to function as a ball-and-socket while moving through an expected range of flexion in the joint. This is also referred to as medial congruent femoral articulation. It should also be appreciated that due to the kinematic behavior inherent in a medial pivot, the design process for medial articular surface 520 on bearing insert 500 may not be repeated on lateral articular surface 530 in many cases, since movement of a point of contact between the lateral condyle of femoral component 600 and lateral articular surface 530 on the lateral articular surface is expected to change through a range of flexion angles of the knee.

The result of this design is a bearing insert adapted to facilitate a desired medial pivot between the tibia and the femur through a range of motion of the knee joint. The ball and socket characteristic on the medial side keeps the femur in a similar location on the medial articular surface of the tibia through a range of motion as would be expected for a medial pivot, while the less constricted lateral side allows the femur to move along the lateral articular surface through the range of motion. One advantage of the bearing insert design that results from this method is improved stability in the joint through expected post-surgical wear compared to more traditional implant designs.

It should also be appreciated that upon completion of an alignment of the femoral component with the tibial insert in the sagittal plane as described above, a posterior-most end of condylar surface 610 should be overhanging a posterior-most end of the tibia insert in the sagittal plane by approximately 3mm to approximately 8mm. In many instances, this posterior overhang will be approximately 5mm. Such an overhang has the characteristics of a healthy natural knee joint, and in this additional way, the method reproduces the natural characteristics of the knee joint.

The method of design may be varied in many ways. In instances where the natural kinematics of a patient produce a lateral pivot in the knee, the method may be performed to design a lateral articular surface contour rather than a medial articular surface contour. In other embodiments, an outer profile of the femoral component, the bearing insert, or both, may be modified while the articular surface design is preserved. Put another way, opposing articular surfaces may be kept in alignment with one another while an outer profile, i.e., perimeter shape of the implant component or components may be modified. Such modification may involve angulation of the existing profile about a center of the component. For example, a bearing insert outer profile may be internally rotated relative to an orientation where the insert is aligned with an anterior-posterior alignment, while a femoral component may be externally rotated relative to an orientation where the femoral component is aligned with an anterior-posterior alignment. In one specific example, the bearing insert may be rotated internally approximately 1-3 degrees while the femoral component may be rotated externally approximately 1-3 degrees. Such modification may assist in preserving a functional alignment of the tibia and femur. Further, such modification may also reduce the risk of wear and future injury due to strain on the patella and patellar tendon.

In other examples, the method may be performed with a view to a patient-specific design. Such method may be the same as that already described, with differences such as one or more of the following. For the initial selection of a bearing insert, the articular surface, including the radial arcs, may be determined on a patient-specific basis rather than based on an off-the-shelf design. The femoral component may also be patient-specific. Further, when aligning the femoral component with respect to the bearing insert in a sagittal plane, patient-specific needs may be taken into account for the thickness of the bearing insert.

In other embodiments, the methods of optimizing a design of a bearing insert to obtain medial congruent articulation of the tibia relative to the femur may be employed in combination with methods of selecting a bearing insert size to complement a baseplate size, as described elsewhere in the present disclosure. In one example, a bearing insert is designed to have a medial articular surface having medial congruency with a femoral component. Such bearing insert may have a hole to receive a latch, such as is shown in FIG. 12. With this arrangement, a desired baseplate sized to fit a tibia may be selected for use with the designed bearing insert. As described elsewhere in the present disclosure, one of many sizes of baseplates may be attached to the bearing insert based on the devices, systems and methods associated with the contemplated universal insert-baseplate interface.

As with other aspects of the present disclosure, the aforementioned method of design may further include a step of fabricating a bearing insert and/or other implant components using additive manufacturing techniques. The additive manufacturing techniques employed may be, for example, those described elsewhere in the present disclosure. Moreover, materials used to fabricate the various implant components designed via the method may be those as described for the components of the universal insert-baseplate interface.

According to a first configuration, a modular implant system comprises: a baseplate; a bearing insert including an articular surface and a plate-contacting surface opposite the articular surface; and a latch adapted for disposal through a hole in the bearing insert, wherein the baseplate is adapted to receive the bearing insert, and wherein when the plate-contacting surface of the bearing insert is disposed on the baseplate and the latch is disposed through the hole in the bearing insert, the latch is rotatable from a first position to a second position, the bearing insert being removable from the baseplate when the latch is in the first position, and the bearing insert being held in place on the baseplate when the latch is in the second position.

According to a second configuration, the modular implant system having the first configuration is further configured such that the latch includes a length with a shaft portion and a tab portion extending from the shaft portion, the tab portion having an elongate dimension angled relative to the length of the latch and the elongate dimension being longer than a maximum diameter of the shaft portion.

According to a third configuration, the modular implant system having the second configuration is further configured such that the hole of the bearing insert includes an upper portion extending from the articular surface, a lower portion extending from the plate-contacting surface and a central portion therebetween, the central portion having a smaller cross-sectional dimension than the upper and lower portions.

According to a fourth configuration, the modular implant system having the third configuration is further configured such that the upper and lower portions of the hole include concave wall surfaces and the central portion includes opposing wall sections projecting relative to the concave wall surfaces, the tab portion of the latch fitting in the central portion when elongate side walls of the tab portion are aligned with surfaces of the opposing wall sections of the central portion.

According to a fifth configuration, the modular implant system having the fourth configuration is further configured such that when the latch is disposed in the hole, the opposing wall sections prevent removal of the latch from the bearing insert when the tab portion of the latch is disposed in the lower portion of the hole and the elongate side walls of the tab portion are not aligned with the surfaces of the opposing wall sections of the central portion.

According to a sixth configuration, the modular implant system having any one of the first to third configurations is further configured such that the latch includes a radial projection on a side surface thereof and the hole is defined by an inner wall including a first indentation and a second indentation, the radial projection being positioned in the first indentation in the first position and the radial projection being positioned in the second indentation in the second position.

According to a seventh configuration, the modular implant system having the sixth configuration is further configured such that when the latch is in the second position, the second indentation prevents the latch from being rotated back into the first position.

According to an eighth configuration, the modular implant system having any one of the sixth to seventh configurations is further configured such that the inner wall includes a third indentation located such that the second indentation separates the first indentation and the third indentation, the latch being removable from the bearing insert when the radial projection is positioned in the third indentation, and the latch being irremovable from the bearing insert when the latch is in the first indentation and in the second indentation.

According to a ninth configuration, the modular implant system having any one of the first to eighth configurations is further configured such that the baseplate includes a first protrusion defining a first recess thereunder such that a second protrusion of the bearing insert is slidable into the first recess to partially prevent sliding of the bearing insert relative to the baseplate.

According to a tenth configuration, the modular implant system having any one of the first and sixth to ninth configurations is further configured such that the latch has a length approximately equal to the hole of the bearing insert.

According to an eleventh configuration, the modular implant system having any one of the first to tenth configurations is further configured such that the baseplate is one of a plurality of baseplates, each baseplate of the plurality of baseplates having a different size with respect to the others.

According to a twelfth configuration, the modular implant system having any one of the first to eleventh configurations is configured such that the bearing insert is one of a plurality of bearing inserts, each bearing insert of the plurality of bearing inserts having a different size with respect to the others.

According to a thirteenth configuration, a kit comprises: the modular implant system having the first to tenth configurations, wherein the bearing insert is a first bearing insert; and a second bearing insert adapted for attachment to the baseplate, the second bearing insert having a different size than the first bearing insert.

According to a fourteenth configuration, the kit having the thirteenth configuration is further configured such that the latch is adapted for disposed in a hole of the second bearing insert.

According to a fifteenth configuration, a kit comprises: the modular implant system according to any one of the first to tenth configurations, wherein the baseplate is a first baseplate; and a second baseplate adapted for attachment to the bearing insert, the second baseplate having a different size than the first baseplate.

It should be appreciated that any of the devices, systems, kits and methods disclosed herein may be designed, formed and/or used in conjunction with robotic technology. For example, any of the implant components described herein may be used with robotic surgical systems to implant the component in a patient. The component may be manipulated with a robotic system or a robotic arm to rotate or position the component, and to secure the component to bone or another component during a procedure. Further, any or all of the steps described in the methods for performing an implant placement procedure, including placement of one or more of the implant components, may be performed using a robotic system. Similarly, robotics may be used in methods of forming the implant components. Additionally, additive manufacturing techniques may be used, such as ALM processes, to form implant components, such as the bearing insert, baseplate and femoral component.

Although the disclosure herein has been described with reference to particular embodiments, it is to be understood that these embodiments are merely illustrative of the principles and applications of the present disclosure. It is therefore to be understood that numerous modifications may be made to the illustrative embodiments and that other arrangements may be devised without departing from the spirit and scope of the present disclosure as defined by the appended claims.

## Claims

1. A method of designing a tibial bearing insert comprising:
identifying a radial arc centerline axis of a tibial bearing insert, the radial arc centerline axis passing through an apex of a lateral radial arc of the tibial bearing insert and an apex of a medial radial arc of the tibial bearing insert, both in a first transverse plane, the lateral and medial radial arcs representing lines of minimum thickness of the tibial bearing insert between an anterior side and a posterior side of the tibial bearing insert on an initial articular surface of the tibial bearing insert;
aligning a center axis of a femoral flexion radius of a femoral component with the radial arc centerline axis such that the center axis and the radial arc centerline axis are coincident with a first coronal plane; while keeping the center axis in the first coronal plane, aligning the femoral component with the tibial bearing insert by positioning the femoral component such that an inferior most point of a circle representative of the femoral flexion radius and centered on the center axis defines a predetermined minimum thickness of the tibial bearing insert; and
subsequent to aligning the center axis and aligning the femoral component with the tibial bearing insert, modifying a curvature of the initial articular surface of the tibial bearing insert between the anterior side and the posterior side to a modified articular surface that corresponds to a portion of the circle.

2. The method of claim 1, wherein identifying the apex of the lateral radial arc and the apex of the medial radial arc includes determining a location on the respective radial arcs that is furthest from a central anterior-posterior axis through the tibial bearing insert.

3. The method of any one of claims 1 and 2, further comprising: selecting a baseplate from among a plurality of baseplates having different sizes; and attaching the tibial bearing insert onto the baseplate such that the modified articular surface of the tibial bearing insert faces away from the baseplate.

4. The method of any one of claims 1-3, wherein aligning the femoral flexion radius of a femoral component with the radial arc centerline axis includes aligning a medial condyle of the femoral component with the radial arc centerline axis.

5. The method of any one of claims 1-4, further comprising selecting the tibial bearing insert prior to the identifying step, the tibial bearing insert being selected from among a plurality of tibial bearing inserts having one or more sizes and one or more articular surface profiles.

6. The method of any one of claims 1-5, further comprising determining the initial articular surface of the tibial bearing insert prior to the identifying step by matching the initial articular surface with an anatomy of a patient intended to receive the tibial bearing insert.

7. The method of any one of claims 1-6 wherein the method is performed using a computer system, the computer system including a processor, a memory and a storage device to store data.

8. A kit comprising:
a plurality of tibial bearing inserts and a baseplate,
wherein the plurality of tibial bearing inserts are designed in accordance with the method of any one of claims 1, 2 and 4-7,
wherein at least two tibial bearing inserts among the plurality of bearing inserts have different sized outer perimeters, and
wherein the baseplate is adapted to receive each tibial bearing insert of the plurality of tibial bearing inserts.

9. The kit of claim 8, wherein each tibial bearing insert of the plurality of tibial bearing inserts includes a latch disposed therein, the latch being operable between unlocked and locked positions such that when one tibial bearing insert of the plurality of tibial bearing inserts is disposed on the baseplate, the one tibial bearing insert is held in place with respect to the baseplate when the latch is in the locked position.

10. A non-transitory, computer-readable medium storing one or more instructions that, when executed by a computer system, cause the computer system to:
identify a radial arc centerline axis of a tibial bearing insert, the radial arc centerline axis passing through an apex of a lateral radial arc of the tibial bearing insert and an apex of a medial radial arc of the tibial bearing insert, both in a first transverse plane, the lateral and medial radial arcs representing lines of minimum thickness of the tibial bearing insert between an anterior side and a posterior side of the tibial bearing insert on an initial articular surface of the tibial bearing insert;
align a center axis of a femoral flexion radius of a femoral component with the radial arc centerline axis such that the center axis and the radial arc centerline axis are coincident with a first coronal plane;
while the center axis is maintained in the first coronal plane, align the femoral component with the tibial bearing insert by positioning the femoral component such that an inferior most point of a circle representative of the femoral flexion radius and centered on the center axis defines a predetermined minimum thickness of the tibial bearing insert; and
subsequent to aligning the center axis and aligning the femoral component with the tibial bearing insert, modify a curvature of the initial articular surface of the tibial bearing insert between the anterior side and the posterior side to a modified articular surface that corresponds to a portion of the circle.

11. The non-transitory, computer-readable medium of claim 10, wherein when executed by a computer system, cause the computer to receive data representative of the tibial bearing insert and the femoral component prior to identifying the radial arc centerline axis.
